# FASCICULE DE BREVET EUROPEEN

(11) **EP 1 725 211 B1**
(45) Date de publication et mention de la délivrance du brevet: **26.09.2007**
(21) Numéro de dépôt: 05730818.1
(22) Date de dépôt: 21.02.2005
(51) Int. Cl.: A61K 8/362, A61K 8/368, A61Q 19/08, A61Q 19/02, A61Q 19/00

(54) **PROCEDE COSMETIQUE DE PEELING UTILISANT L ACIDE 8-HEXADECENE -1,16-DICARBOXYLIQUE**
KOSMETISCHES PEELINGVERFAHREN UNTER VERWENDUNG DER 8-HEXADECEN-1,16-DICARBONSÄURE
PEELING COSMETIC METHOD USING A 8-HEXADECENE-1,16-DICARBOXYLIC ACID

(30) Priorité: 19.02.2004 FR 0450307; 05.05.2004 US 567788 P
(43) Date de publication de la demande: 29.11.2006
(73) Titulaire: L'ORÉAL, 75008 Paris (FR)
(72) Inventeur: HANSENNE, Isabelle, 07090 Westfield, NJ (US); SORE, Gabrielle, F-75012 Paris (FR)
(74) Mandataire: Leonard, Armelle
(86) Numéro de dépôt international: PCT/FR2005/000399
(87) Numéro de publication internationale: WO 2005/089707

(56) Documents cités:
- DE-A- 10 150 734
- FR-A- 2 754 253
- UNIQEMA: "Implications of a new mechanism of action for Arlatone Dioic DCA (octadecenedioic acid) and possible synergies with other molecules that bind to the peroxisomal proliferator activated receptor" RESEARCH DISCLOSURE, KENNETH MASON PUBLICATIONS, HAMPSHIRE, GB, vol. 477, no. 2, janvier 2004 (2004-01), XP007133262 ISSN: 0374-4353
- ANONYMOUS: "ARLATONE DIOIC DCA : Beautiful skin for every body" INTERNET ARTICLE, [Online] 21 avril 2003 (2003-04-21), XP002301860 Extrait de l'Internet: URL:http://www.uniqema.com/pc/possibility3 .htm> [extrait le 2004-10-21]

## Description

La présente invention se rapporte à un procédé de traitement cosmétique des irrégularités visibles et/ou tactiles de la peau, destiné en particulier à atténuer les rides et ridules et/ou les taches pigmentaires et/ou les cicatrices telles que les marques d'acné ou de varicelle, comprenant les étapes consistant à : (a) appliquer topiquement sur la peau d'une composition renfermant, dans un milieu physiologiquement acceptable, au moins 10% en poids d'acide 8-hexadécène-1,16-dicarboxylique, (b) laisser la composition au contact de la peau pendant une durée allant de 5 mn à 6 heures, et (c) éliminer la composition par rinçage.

L'acide 8-hexadécène-1,16-dicarboxylique ou acide 9-octadécène dioïque est un composé qui se trouve de façon prédominante sous forme cis, obtenu par biofermentation de l'acide oléique en présence d'une levure mutante de l'espèce *Candida*. Il présente notamment des propriétés blanchissantes et anti-microbiennes permettant d'envisager son utilisation dans des produits déodorants, anti-pelliculaires et anti-acné, comme décrit par J. W. WIECHERS et al. dans Cosmetics & Toiletries, Vol. 117, n° 7, p.55-68 (Juillet 2002) et dans SÖFW Journal, 128, p.2-8 (2002).

Il a en outre été proposé dans la demande WO 03/032941 de l'utiliser dans des compositions destinées à empêcher le brunissement de la peau, en association avec des agents anti-oxydants. Son utilisation comme dépigmentant de substitution à l'acide kojique ou à l'hydroquinone, en particulier dans des compositions de nettoyage, a également été suggérée dans le document DE-101 50 734.

Toutefois, à la connaissance de la Demanderesse, il n'a encore jamais été proposé d'utiliser ce composé pour réaliser des peelings.
Les peelings chimiques sont un moyen bien connu pour améliorer l'aspect de surface de la peau, en particulier pour atténuer des défauts de pigmentation tels que les lentigo actiniques ou les marques d'acné ou de varicelle, ou pour lisser les irrégularités de la texture de la peau, en particulier les rides et ridules, en provoquant une destruction limitée de l'épiderme et des couches superficielles du derme.

Il a déjà été suggéré d'utiliser divers composés seuls ou en association, et en particulier l'acide glycolique et les acides de fruits, la résorcine, l'acide trichloracétique, le phénol et l'acide rétinoïque, pour réaliser des peelings chimiques. Toutefois, bien que ces composés aient pu donner des résultats satisfaisants, il n'en reste pas moins qu'il subsiste le besoin de disposer de compositions de peeling qui soient efficaces tout en étant bien tolérées.

Il est maintenant apparu à la Demanderesse que l'acide 8-hexadécène-1,16-dicarboxylique pouvait permettre la réalisation de peelings chimiques efficaces et bien tolérés.

La présente invention a donc pour objet un procédé de traitement cosmétique des irrégularités visibles et/ou tactiles de la peau, comprenant les étapes consistant à :
(a) appliquer topiquement sur la peau d'une composition renfermant, dans un milieu physiologiquement acceptable, au moins 10% en poids d'acide 8-hexadécène-1,16-dicarboxylique,
(b) laisser la composition au contact de la peau pendant une durée allant de 5 mn à 6 heures, et
(c) éliminer la composition par rinçage.

L'acide 8-hexadécène-1,16-dicarboxylique utilisé selon l'invention peut être sous forme cis, sous forme trans, ou sous un mélange de ces deux formes. Il est notamment disponible dans le commerce auprès de la société UNIQEMA sous la dénomination commerciale Arlatone Dioic DCA.

La quantité d'acide 8-hexadécène-1,16-dicarboxylique utilisé selon l'invention dépend du résultat recherché et en particulier de la profondeur du peeling que l'on cherche à obtenir, qui est elle-même fonction de la condition de la peau à améliorer. Pour donner un ordre de grandeur, la quantité d'acide 8-hexadécène-1,16-dicarboxylique peut représenter de 10 à 50%, et de préférence de 15 à 35%, du poids de la composition.

La composition utilisée selon l'invention est adaptée à une application topique sur la peau et comprend donc généralement un milieu physiologiquement acceptable, c'est-à-dire compatible avec la peau et éventuellement avec ses phanères.

Elle peut se présenter sous toutes les formes galéniques classiquement utilisées pour une application topique, pour autant qu'elle s'élimine facilement par rinçage, et notamment sous forme de gel ou de solution hydroglycolique ou hydroalcoolique. Elle peut aussi, par ajout d'une phase grasse ou huileuse, se présenter sous forme de dispersion du type lotion ou d'émulsion de consistance liquide ou semi-liquide, obtenue de préférence par dispersion d'une phase grasse dans une phase aqueuse (H/E). En variante, la composition selon l'invention peut se présenter sous forme de masque. Ces compositions sont préparées selon les méthodes usuelles.

Lorsque la composition est sous forme d'émulsion, la proportion de la phase huileuse de l'émulsion peut aller par exemple de 1 à 30 % en poids, et de préférence de 5 à 20 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique
ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Comme matières grasses utilisables dans l'invention, on peut utiliser les huiles et notamment les huiles minérales (huile de vaseline), les huiles d'origine végétale (huile d'avocat, huile de soja), les huiles de synthèse (perhydrosqualène), les huiles siliconées (cyclométhicone) et les huiles fluorées (perfluoropolyéthers).

Comme émulsionnants et coémulsionnants utilisables dans l'invention, on peut citer par exemple les éthers d'alcool gras et de polyalkylène glycol tels que l'éther stéarylique oxypropyléné (15 PPG) et les éthers stéaryliques oxyéthylénés (2 et 21 OE, notamment).

La composition selon l'invention peut également contenir les adjuvants habituels dans les domaines cosmétique et dermatologique, tels que les épaississants, les actifs, les conservateurs, les solvants, et les charges. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse ou dans la phase aqueuse. Ces adjuvants, ainsi que leurs concentrations, doivent être tels qu'ils ne nuisent pas aux propriétés de l'acide 8-hexadécène-1,16-dicarboxylique.

Comme épaississants, on peut citer en particulier : la gomme de xanthane, un homo- ou copolymère d'acide acrylique éventuellement réticulé, un polyacrylamide, un homo- ou copolymère d'acide acrylamido méthylpropane sulfonique, et les dérivés de cellulose dont l'hydroxypropyl cellulose.

La composition renferme avantageusement au moins un composé choisi parmi l'éthanol, le propylène glycol, le dipropylène glycol, l'acide isostéarique, l'alcool isostéarique, l'isostéarate de propylène glycol, l'isostéarate de glycéryle et le diméthyl isosorbide qui améliorent la solubilité de l'acide 8-hexadécène-1,16-dicarboxylique.

En outre, selon une forme d'exécution préférée, la composition utilisée selon l'invention renferme au moins un β-hydroxyacide.

Ainsi, cette composition peut comprendre, dans un milieu physiologiquement acceptable adapté à une application topique sur la peau : (a) de 10 à 50% en poids d'acide 8-hexadécène-1,16-dicarboxylique, et (b) de 0,1 à 15% en poids, de préférence de 0,2 à 10% en poids, d'au moins un β-hydroxyacide, par rapport au poids total de la composition.

Comme β-hydroxyacides, on peut citer l'acide salicylique et ses dérivés, en particulier les composés de formule (1) suivante ou un sel d'un tel dérivé : 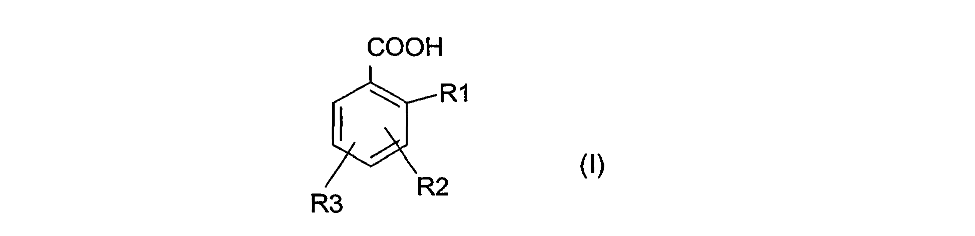 dans laquelle :
- R₁ représente un radical hydroxyle ou un ester de formule :

   -O-CO-R₄

   dans laquelle R₄ est un radical aliphatique, saturé ou insaturé, comprenant de 1 à 26 atomes de carbone, et de préférence de 1 à 18 atomes de carbone, une fonction amine ou thiol éventuellement substituée par un radical alkyle comprenant de 1 à 18 atomes de carbone, et de préférence de 1 à 12 atomes de carbone,
- R₂ et R₃ indépendamment l'un de l'autre se trouvent en position 3, 4, 5 ou 6 sur le noyau benzénique et représentent indépendamment l'un de l'autre, un atome d'hydrogène ou un radical :

   -(O)ₙ-(CO)ₘ-R₅
dans lequel n et m, indépendamment l'un de l'autre, sont chacun un nombre entier égal à 0 ou 1 ; à la condition que R₂ et R₃ ne soient pas simultanément des atomes d'hydrogène ;
- R₅ représente un atome d'hydrogène, un radical aliphatique saturé comprenant de 1 à 18 atomes de carbone, linéaire, ramifié ou cyclisé, un radical insaturé comprenant de 3 à 18 atomes de carbone, portant une à neuf doubles liaisons conjuguées ou non, les radicaux pouvant être substitués par au moins un substituant choisi parmi les atomes d'halogène (fluor, chlore, brome, iode), les radicaux trifluorométhyle, hydroxyle sous forme libre ou estérifiée par un acide comprenant de 1 à 6 atomes de carbone, ou carboxyle libre ou estérifié par un alcool inférieur comprenant de 1 à 6 atomes de carbone.

De manière préférée, le dérivé d'acide salicylique de formule (I) est tel que R₁ représente un radical hydroxyle, R₂ représente un atome d'hydrogène, R₃ est en position 5 du noyau benzénique et représente un radical -CO-R₅ où R₅ représente un radical aliphatique saturé comprenant de 3 à 15 atomes de carbone.

Selon un mode de réalisation préféré de l'invention, le dérivé d'acide salicylique de formule (I) est choisi parmi les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique, 5-tert-octylsalicylique, 3-tert-butyl-5-méthylsalicylique, 3-tert-butyl-6-méthylsalicylique, 3,5-diisopropylsalicylique, 5-butoxysalicylique, 5-octyloxysalicylique, propanoyl-5-salicylique, n-hexadecanoyl-5-salicylique, n-oléoyl-5-salicylique, benzoyl-5-salicylique, leurs sels monovalents et divalents et leurs mélanges. Il s'agit plus particulièrement de l'acide n-octanoyl-5-salicylique (nom INCI : Capryloyl salicylic Acid).

La quantité de β-hydroxyacide peut représenter de 0,1 à 15%, et de préférence de 0,2 à 10%, du poids total de la composition.

En variante ou en plus, la composition utilisée selon l'invention peut comprendre au moins un agent desquamant choisi notamment parmi : les α-hydroxyacides tels que les acides glycolique, citrique, lactique, tartrique, malique ou mandélique ; l'acide ascorbique et ses dérivés tels que le glucoside d'ascorbyle et l'ascorbyl phosphate de magnésium ; la nicotinamide ; l'urée ; et l'acide (N-2 hydroxyéthylpiperazine-N-2-éthane) sulfonique (HEPES).

Comme indiqué précédemment, le procédé selon l'invention est destiné à être utilisé pour réaliser un peeling chimique superficiel visant à atténuer les irrégularités visibles et/ou tactiles de la peau, et en particulier à atténuer les rides et ridules et/ou les taches pigmentaires et/ou les cicatrices. Il est donc de préférence mis en oeuvre sur des personnes présentant de l'acné et/ou des rides et/ou des cicatrices et/ou des défauts de pigmentation tels que des mélasmas et des lentigos séniles ou actiniques.

Ce procédé peut notamment être mis en oeuvre par une esthéticienne.

Selon ce procédé, la composition utilisée selon l'invention peut être appliquée sur le visage et/ou le cou et/ou le décolleté et/ou les mains et/ou le dos par tout moyen permettant une répartition uniforme et notamment à l'aide d'un coton, d'une tige, d'un pinceau, d'une gaze, d'une spatule ou d'un tampon, ou encore par pulvérisation, et peut être éliminée par rinçage à l'eau ou à l'aide d'un détergent doux après être restée au contact de la peau pendant une durée comprise entre 5 mn et 6 heures, de préférence entre 5 mn et 30 mn.

Pour optimiser ses effets, le procédé de peeling comprend de préférence des étapes additionnelles de préparation de la peau au peeling (pour améliorer l'efficacité et l'homogénéité du peeling) et/ou de soin de la peau après peeling à l'aide de compositions renfermant de plus faibles quantités d'acide 8-hexadécène-1,16-dicarboxylique que la composition décrite précédemment.

Les compositions utilisées dans ces étapes préliminaire et supplémentaire peuvent être appliquées matin et soir, par exemple, éventuellement en association avec une composition destinée à protéger la peau contre les effets des UV. La composition de pré-traitement peut être appliquée pendant une à quatre semaines et la composition de post-traitement pendant un jour à huit semaines, par exemple.

Le procédé de peeling ci-dessus, y compris les étapes préliminaire et supplémentaire éventuelles, peut être mis en oeuvre une seule fois ou renouvelé jusqu'à cinq fois, si nécessaire. La fréquence d'application est comprise entre deux fois par semaine et une fois toutes les trois semaines. Elle est de préférence d'une application par semaine.

L'invention sera maintenant illustrée par les exemples non limitatifs suivants. Dans ces exemples, les quantités sont indiquées en pourcentage pondéral.

### EXEMPLES

### Exemple 1 : Evaluation in vivo de l'effet de l'acide 8-hexadécène-1,16-dicarboxylique

### Protocole

On a évalué l'efficacité de l'acide 8-hexadécène-1,16-dicarboxylique sur un panel de 12 femmes de type caucasien, âgées de 55 à 65 ans, présentant des signes de photo-vieillissement sur les avant-bras.

Pour ce faire, on a appliqué une Composition A renfermant 10% d'acide 8-hexadécène-1,16-dicarboxylique sur une zone de 9 cm² située sur la face antérieure des avant-bras des sujets, à raison de 15 mg/cm². La solution a été laissée 5 minutes sur la zone d'application avant d'être retirée à l'aide d'une compresse imbibée d'eau tiède. Le traitement a été réalisé une fois par jour pendant cinq jours consécutifs.

Une évaluation de la tolérance (irritation et sensations subjectives) a été réalisée après chaque application. Des mesures non invasives ont en outre permis de mesurer la perte insensible en eau (PIE), la couleur de la peau (système L*a*b*), l'élasticité cutanée (par cutométrie) et le microrelief cutané (par prise d'empreintes), à To et au bout de 8 jours.

La PIE a précisément été mesurée à l'aide d'un Tewamètre (Courage et Khazaka), la mesure du taux d'évaporation cutanée, exprimée en g/m²/h étant prise après stabilisation de la valeur, c'est-à-dire environ une minute après la pose de la sonde sur la surface de peau à analyser. Les mesures colorimétriques ont été réalisées à l'aide d'un chromamètre CR 300 (MINOLTA) puis transférées vers un ordinateur de type PC. Les mesures d'élasticité ont été réalisées à l'aide d'un Cutomètre (Courage et Khazaka). Les prises d'empreintes ont été réalisées en utilisant un mastic élastomère SILFLO puis un système d'analyse d'images destiné à mesurer : la surface moyenne des creux, leur longueur moyenne, leur profondeur moyenne et leur facteur de forme.

La Composition A a été comparée à une Composition B, appliquée dans les mêmes conditions (sur un autre site de l'avant-bras de chacun des sujets) et renfermant 30% d'acide glycolique dans 60% d'eau et 10% de glycérine, utilisée à titre de composition de référence. Après application de cette composition, la zone de peau traitée a toutefois été neutralisée et rincée pour apaiser les picotements.

Une zone de peau de l'avant-bras non traitée a été utilisée comme témoin pour chaque sujet.

Les compositions A et B ont été comparées par un test de Student pour données appariées. Le taux de significativité des tests a été fixé à p < 0.05.

### Résultats

Les résultats du test de PIE sont rassemblés dans le Tableau ci-dessous :

| Composition | PIE à To (g/m²/h) | PIE au 8^{ème} jour (g/m²/h) | Différence (J8 - J1) | Variation par rapport au témoin |
|---|---|---|---|---|
| A | 9.35 | 9.46 | 0.11 | 0.91 |
| B | 9.45 | 8.78 | -0.67 | 0.14 |
| Site témoin | 9.45 | 8.65 | -0.81 | 0.00 |

Comme il ressort de ce tableau, l'application de la composition A contenant l'acide 6-hexadécène-1,16-dicarboxylique a entraîné une augmentation de la Perte Insensible en Eau de la peau, plus importante que celle observée avec l'acide glycolique qui est un actif de peeling de référence. Ces résultats suggèrent que ce composé est un bon candidat comme actif de peeling.

### Exemple 2 : Composition de peeling anti-rides

On prépare la composition ci-dessous de façon classique pour l'homme du métier.

| | | |
|---|---|---|
| Acide 8-hexadécène-1,16-dicarboxylique | | 15 % |
| Acide mandélique | | 10 % |
| Eau | | 20 % |
| Polyéthylène glycol | qsp | 100 % |

Cette composition peut être appliquée sous forme de peeling pour atténuer les rides et ridules faciales.

### Exemple 3 : Composition de peeling blanchissant

On prépare la composition ci-dessous de façon classique pour l'homme du métier.

| | | |
|---|---|---|
| Acide 8-hexadécène-1,16-dicarboxylique | | 15 % |
| Calcium D-pantéthéine sulfonate | | 1 % |
| Eau | | 15 % |
| Ethanol | qsp | 100 % |

Cette composition peut être appliquée sous forme de peeling pour atténuer les dyschromies (lentigos actiniques ou séniles, mélasmas).

### Exemple 4 : Composition de peeling anti-acné

On prépare la composition ci-dessous de façon classique pour l'homme du métier.

| | | |
|---|---|---|
| Acide 8-hexadécène-1,16-dicarboxylique | | 40 % |
| Acide n-octanoyl-5-salicylique | | 2 % |
| Ethanol | qsp | 100 % |

Cette composition peut être appliquée sous forme de peeling pour atténuer les marques d'acné.

## Revendications

1. Procédé de traitement cosmétique des irrégularités visibles et/ou tactiles de la peau, comprenant les étapes consistant à :
(a) appliquer topiquement sur la peau d'une composition renfermant, dans un milieu physiologiquement acceptable, au moins 10% en poids d'acide 8-hexadécène-1,16-dicarboxylique,
(b) laisser la composition au contact de la peau pendant une durée allant de 5 mn à 6 heures, et
(c) éliminer la composition par rinçage.

2. Procédé selon la revendication 1, **caractérisé en ce que** la composition est laissée au contact de la peau, dans l'étape (b), pendant une durée allant de 5 mn à 30 mn.

3. Procédé selon la revendication 1 ou 2, **caractérisé en ce qu'**il est destiné à atténuer les rides et ridules et/ou les taches pigmentaires et/ou les cicatrices.

4. Procédé selon la revendication 3, **caractérisé en ce que** lesdites cicatrices sont des marques d'acné ou de varicelle.

5. Procédé selon l'une quelconque des revendications 1 à 4, **caractérisée en ce qu'**il est mis en oeuvre sur des personnes présentant de l'acné et/ou des rides et/ou des cicatrices et/ou des défauts de pigmentation.

6. Procédé selon l'une quelconque des revendications 1 à 5, **caractérisé en ce que** la quantité d'acide 8-hexadécène-1,16-dicarboxylique représente de 10 à 50 % du poids total de la composition.

7. Procédé selon la revendication 6, **caractérisé en ce que** la quantité d'acide 8-hexadécène-1,16-dicarboxylique représente de 15 à 35% du poids total de la composition.

8. Procédé selon l'une quelconque des revendications 1 à 7, **caractérisé en ce que** la composition comprend, dans un milieu physiologiquement acceptable adapté à une application topique sur la peau : (a) de 10 à 50% en poids d'acide 8-hexadécène-1,16-dicarboxylique, et (b) de 0,1 à 15% en poids d'au moins un β-hydroxyacide, par rapport au poids total de la composition.

9. Procédé selon la revendication 8, **caractérisé en ce que** ledit β-hydroxyacide est choisi parmi l'acide salicylique et ses dérivés, ou un sel d'un tel dérivé.

10. Procédé selon la revendication 9, **caractérisé en ce que** ledit dérivé d'acide salicylique est l'acide n-octanoyl-5-salicylique.

11. Procédé selon l'une quelconque des revendications 8 à 10, **caractérisé en ce que** la quantité de β-hydroxyacide représente de 0,2 à 10% du poids total de la composition.

## Claims

1. Cosmetic process for treating visible and/or tactile skin irregularities, comprising the steps consisting in:
(a) topically applying to the skin a composition containing, in a physiologically acceptable medium, at least 10% by weight of 8-hexadecene-1,16-dicarboxylic acid,
(b) leaving the composition in contact with the skin for a time ranging from 5 minutes to 6 hours, and
(c) removing the composition by rinsing.

2. Process according to Claim 1, **characterized in that** the composition is left in contact with the skin, in step (b), for a time ranging from 5 minutes to 30 minutes.

3. Process according to Claim 1 or 2, **characterized in that** it is intended for attenuating wrinkles and fine lines and/or pigmentary marks and/or scars.

4. Process according to Claim 3, **characterized in that** the said scars are acne or chickenpox marks,

5. Process according to any one of Claims 1 to 4, **characterized in that** it is performed on individuals with acne and/or wrinkles and/or scars and/or pigmentation defects.

6. Process according to any one of Claims 1 to 5, **characterized in that** the amount of 8-hexadecene-1,16-dicarboxylic acid represents from 10% to 50% of the total weight of the composition.

7. Process according to Claim 6, **characterized in that** the amount of 8-hexadecene-1,16-dicarboxylic acid represents from 15% to 35% of the total weight of the composition.

8. Process according to any one of Claims 1 to 7, **characterized in that** the composition comprises, in a physiologically acceptable medium suitable for topical application to the skin: (a) from 10% to 50% by weight of 8-hexadecene-1,16-dicarboxylic acid and (b) from 0.1% to 15% by weight of at least one β-hydroxy acid, relative to the total weight of the composition.

9. Process according to Claim 8, **characterized in that** the said β-hydroxy acid is chosen from salicylic acid and derivatives thereof, or a salt of such a derivative.

10. Process according to Claim 9, **characterized in that** the said salicylic acid derivative is 5-n-octanoyl-salicylic acid.

11. Process according to any one of Claims 8 to 10, **characterized in that** the amount of β-hydroxy acid represents from 0.2% to 10% relative to the total weight of the composition.

## Patentansprüche

1. Verfahren zur kosmetischen Behandlung von sichtbaren und/oder fühlbaren Unregelmäßigkeiten der Haut, das die folgenden Schritte umfasst:
(a) auf die Haut topisch eine Zusammensetzung aufzutragen, die in einem physiologisch akzeptablen Medium mindestens 10 Gewichtsprozent 8-Hexadecen-1,16-dicarbonsäure enthält,
(b) die Zusammensetzung während einer Zeitdauer von 5 Minuten bis 6 Stunden mit der Haut in Kontakt zu belassen, und
(c) die Zusammensetzung durch Spülen abzunehmen.

2. Verfahren nach Anspruch 1, **dadurch gekennzeichnet, dass** die Zusammensetzung in Schritt (b) während einer Zeitdauer von 5 bis 30 min mit der Haut in Kontakt belassen wird.

3. Verfahren nach Anspruch 1 oder 2, **dadurch gekennzeichnet, dass** es dazu dienen soll, Falten und Fältchen und/oder Pigmentflecken und/oder Narben abzumildern.

4. Verfahren nach Anspruch 3, **dadurch gekennzeichnet, dass** es sich bei den Narben um Aknenarben oder Windpockennarben handelt.

5. Verfahren nach einem der Ansprüche 1 bis 4, **dadurch gekennzeichnet, dass** es bei Personen durchgeführt wird, die Akne und/oder Falten und/oder Narben und/oder Pigmentierungsstörungen haben.

6. Verfahren nach einem der Ansprüche 1 bis 5, **dadurch gekennzeichnet, dass** der Mengenanteil der 8-Hexadecen-1,16-dicarbonsäure 10 bis 50 % des Gesamtgewichts der Zusammensetzung ausmacht.

7. Verfahren nach Anspruch 6, **dadurch gekennzeichnet, dass** der Mengenanteil der 8-Hexadecen-1,16-dicarbonsäure 15 bis 35 % des Gesamtgewichts der Zusammensetzung ausmacht.

8. Verfahren nach einem der Ansprüche 1 bis 7, **dadurch gekennzeichnet, dass** die Zusammensetzung in einem physiologisch akzeptablen, für eine topische Anwendung auf die Haut geeigneten Medium enthält; (a) 10 bis 50 Gew.-% 8-Hexadecen-1,16-dicarbonsäure und (b) 0,1 bis 15 Gew.-% mindestens einer β-Hydroxysäure, bezogen auf das Gesamtgewicht der Zusammensetzung.

9. Verfahren nach Anspruch 8, **dadurch gekennzeichnet, dass** die β-Hydroxysäure unter Salicylsäure und ihren Derivaten oder einem Salz eines solchen Derivates ausgewählt ist.

10. Verfahren nach Anspruch 9, **dadurch gekennzeichnet, dass** es sich bei dem Salicylsäurederivat um 5-n-Octanoylsalicylsäure handelt.

11. Verfahren nach einem der Ansprüche 8 bis 10, **dadurch gekennzeichnet, dass** der Mengenanteil der β-Hydroxysäure 0,2 bis 10 % des Gesamtgewichts der Zusammensetzung beträgt.
